# EUROPEAN PATENT APPLICATION

(11) **EP 4 174 469 A1**
(43) Date of publication of application: **03.05.2023**
(21) Application number: 21832355.8
(22) Date of filing: 30.06.2021
(51) Int. Cl.: G01N 1/10, G01N 33/00

(54) **HAZARDOUS CHEMICAL COLLECTION APPARATUS AND GAGE SYSTEM**

(30) Priority: 30.06.2020 CN 202010616691
(71) Applicant: Nuctech Company Limited, TongFang Building Shuangqinglu Haidian District Beijing 100084 (CN); Tsinghua University, Beijing 100084 (CN)
(72) Inventor: CHEN, Zhiqiang, Beijing 100084 (CN); ZHANG, Li, Beijing 100084 (CN); HUANG, Qingping, Beijing 100084 (CN); DING, Hui, Beijing 100084 (CN); QIU, Quanyuan, Beijing 100084 (CN); ZHOU, Yong, Beijing 100084 (CN); CHEN, Jiabao, Beijing 100084 (CN); BAO, Yuntai, Beijing 100084 (CN); ZHANG, Lei, Beijing 100084 (CN)
(74) Representative: Cabinet Beau de Loménie
(86) International application number: PCT/CN2021/103521
(87) International publication number: WO 2022/002126

(57) **Abstract**

The present disclosure relates to a hazardous chemical collection apparatus and a gate system. The hazardous chemical collection apparatus comprises a vibration portion (2) and a collection portion (3). The vibration portion (2) applies a vibration to an access credential (11) such that hazardous chemical attached onto the surface of the access credential (11) falls off. The collection portion (3) is positioned below the access credential (11) to collect the hazardous chemical falling off from the access credential (11). The gate system comprises the hazardous chemical collection apparatus. The hazardous chemical collection apparatus provided in the present application can automatically complete a hazardous chemical collection, is not easily perceived by a passing person, saves cost of sampling test papers, has considerable economic benefit, reduces the workload of security screeners, and reduces human resources management cost.

## Description

### CROSS REFERENCES TO RELATED APPLICATIONS

The present disclosure claims priority to Chinese Patent Application No. 202010616691.3, filed June 30, 2020 and titled "HAZARDOUS CHEMICAL COLLECTION APPARATUS AND GATE SYSTEM", the content of which is incorporated herein by reference in its entirety.

### TECHNICAL FIELD

The present disclosure relates to a technical field of security inspection, and specifically relates to a hazardous chemical collection apparatus and a gate system.

### BACKGROUND

In recent years, in airports, railway stations, subways, large venues, buildings, etc., in order to ensure safety of people and facilities, it is necessary to carry out security inspection on the human bodies and carried goods, to avoid hazardous chemicals, such as inflammable and explosive articles and drugs, from being brought into the above-mentioned places.

At present, in airport and other facilities, test paper is generally used to manually wipe surfaces of clothes, passengers' pockets and other parts for sampling.

### SUMMARY

The sampling by test paper as described above is easily perceived by the passing person and tends to attract the attention of suspicious persons carrying hazardous chemicals such as the drugs. Further, the process is tedious, the work intensity of security inspectors is high, the passenger experience is poor, and the efficiency is low with a high labor cost. Moreover, the consumption of the test paper for wiping is large, resulting in high sampling cost.

The present disclosure provides a hazardous chemical collection apparatus which can take an access voucher as an object.

One implementation of the present disclosure provides a hazardous chemical collection apparatus including a vibration unit and a collection unit, the vibration unit applies vibrations to an access voucher to cause hazardous chemicals attached to a surface of the access voucher to fall off, and the collection unit is located below the access voucher to collect the hazardous chemicals falling off from the access voucher.

A further implementation of the present disclosure provides a gate system which includes a hazardous chemical collection apparatus as described above.

According to the present disclosure, the hazardous chemicals can be collected by applying vibrations to the access voucher by taking the access voucher as an object. Therefore, compared with the situation of collection with test paper, it is not easy to be perceived by the passing person, can save cost of test paper for sampling, and achieve considerable economic benefits. In addition, as long as the passing person provides the access voucher, the collection apparatus can automatically complete the collection of hazardous chemicals, reducing the workload of security inspector and reducing the cost of human management.

### BRIEF DESCRIPTION OF THE DRAWINGS

Fig. 1 is a brief composition diagram showing an example of Embodiment 1 of the hazardous chemical collection apparatus of the present disclosure.
Fig. 2 is a brief composition diagram showing an example of Embodiment 1 of the hazardous chemical collection apparatus of the present disclosure.
Fig. 3 is a brief composition diagram showing an example of Embodiment 2 of the hazardous chemical collection apparatus of the present disclosure.

### Reference numerals

1 Access voucher support unit
12 Access voucher identification mechanism
13 Access voucher insertion port
14 Support base
2 Vibration unit
21 Electromagnet
22 Rotating shaft
23 Striking head
24 Electromagnet holding bracket
3 Collection unit
4 Air blowing pipe
5 Air suction pipe
51 Three-way connector
6 Hazardous chemical detection device
7 Air pump

### DETAILED DESCRIPTION

Below, the structure of the hazardous chemical collection apparatus of the present disclosure is described based on the accompanying drawings.

The hazardous chemical collection apparatus of the present disclosure is used to collect hazardous chemicals. Here, the hazardous chemicals include at least one of inflammable and explosive articles and drugs. The inflammable and explosive articles may include, but not limited to, alcohol, gasoline, trinitrotoluene (TNT), hexogen (RDX), black powder (BP), pentaerythritol tetranitrate (PENT), nitroglycerin (NG) and ammonium nitrate (AN). The drugs may include, but not limited to, opium, heroin, methamphetamine, morphine, cannabis, cocaine, K powder and ecstasy. Here, the hazardous chemicals can be solid particles or liquids.

In the present disclosure, an access voucher is used as an object of hazardous chemical sampling. This is because, usually, during the process of production and handling, there may be a trace of hazardous chemicals left on the hands of the carriers of hazardous chemicals, and then rubbed on their clothes, pockets, access vouchers, and the like. Therefore, the hazardous chemicals that meet the detection sensitivity of the hazardous chemical detection device can be collected by taking the access voucher as the object for collection. For example, drug addicts may have drug powder on their hands or drugs in their sweat, which may stick to surfaces of the access vouchers when the drug addicts take the access vouchers, so that the drugs can be collected through the access vouchers.

In the present disclosure, the access voucher refers to any bills and certificates that represent the access authority or identity of the passing person, such as a train ticket, an air ticket, a subway ticket, a gate ticket, an access card, an identity card, a passport, a driver's license, and the like.

### (Embodiment 1)

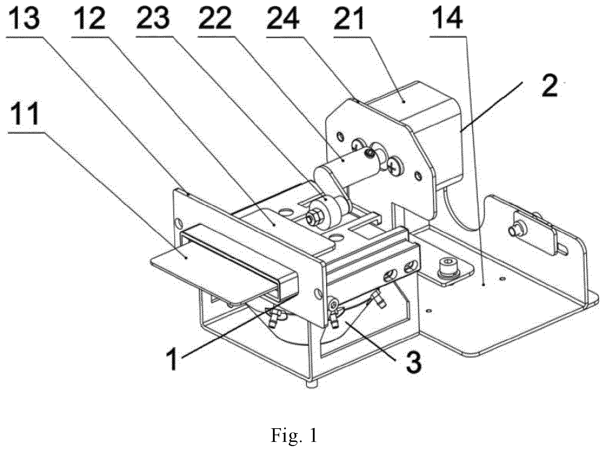

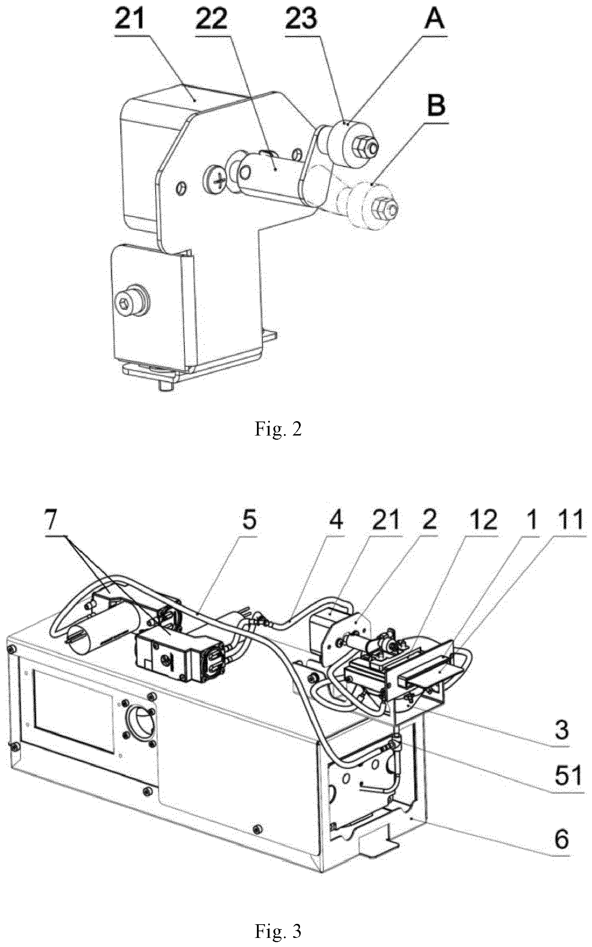

Fig. 1 is a brief composition diagram showing an example of an embodiment of the hazardous chemical collection apparatus in the present disclosure, and it shows the situation that the access voucher 1 as a piece under detection is inserted into the hazardous chemical collection apparatus. As shown in Fig. 1, in the Embodiment 1, the hazardous chemical collection apparatus includes: an access voucher support unit 1, a vibration unit 2, and a collection unit 3 (which may be referred to Fig. 3 of Embodiment 2 as described below). The vibration unit 2 is a part capable of applying vibrations to the access voucher. The collection unit 3 is a part capable of collecting the hazardous chemicals falling off from the access voucher. The collection unit 3 is located below the vibration unit 2 and the access voucher support unit 1, and in this case, the access voucher can be supported by the access voucher support unit 1, and the access voucher support unit 1 can be also used by an access voucher information collection apparatus described later. The collection unit 3 is connected with a hazardous chemical detection device, which is used to detect and analyze the collected hazardous chemicals. The collection unit 3 may be a collection part which has a longitudinal section with a large upper portion and a small lower portion (for example, in a tapered shape). The access voucher support unit 1 can be an access voucher supporting bracket, an access voucher insertion port, or the like, as long as it can support the access voucher. Here, the access voucher refers to any ticket and certificate that represents the access authority or identity of the passing person, such as a train ticket, an air ticket, a subway ticket, a gate ticket, an access card, an ID card, a passport, a driver's license, and the like.

The vibration unit 2 includes a driving mechanism and a moving mechanism. The driving mechanism is connected with the moving mechanism, and drives the moving mechanism to move in translation or rotation. The moving mechanism applies vibrations to the access voucher during the movement.

For example, when the driving mechanism drives the moving mechanism to move between a first limit position away from the access voucher and a second limit position close to the access voucher, vibrations are applied to the access voucher, causing the hazardous chemicals attached to surface(s) of the access voucher to fall off.

The driving mechanism can be an electromagnet, a motor, and the like. The electromagnet here can be a translation electromagnet or a rotary electromagnet, as long as it can make the moving mechanism move between the first limit position and the second limit position.

The moving mechanism can be, for example, but not limited to a striking portion that strikes the access voucher, or a shaking portion that shakes the access voucher, as long as it can apply vibrations to the access voucher during movement. For example, the striking portion can be a hammer, which strikes and thus applies vibrations to the access voucher when moving from the first limit position to the second limit position. For example, the shaking portion can be a clamping member, which shakes and thus applies vibrations to the access voucher when moving back and forth between the first limit position and the second limit with the access voucher being clamped by it.

The first limit position and the second limit position can be defined in advance according to a distance between the moving mechanism and components of the access voucher support unit, such as the access voucher supporting bracket, as long as it can be ensured that other peripheral structures will not be touched. In other words, the relative position between the vibration unit 2 and the access voucher support unit 1 is stationary, and specifically, the relative positions between the first limit position, the second limit position and the access voucher support unit 1 are stationary.

Hereinafter, an example of the Embodiment 1 will be described with reference to Fig. 1 and Fig. 2.

The access voucher support unit 1 includes an access voucher identification mechanism 12, an access voucher insertion port 13 and a support base 14, the access voucher support unit 1 is connected with an electromagnet holding bracket 24 of the vibration unit 2 through the support base 14, so that the position of the access voucher support unit 1 is stationary relative to the vibration unit 2.

The access voucher 11 is inserted into the access voucher insertion port 13, and the access voucher identification mechanism 12 identifies the access voucher 11. The access voucher identification mechanism 12 can detect that an access voucher is inserted into the access voucher insertion port 13, and also can identify the access voucher information on the access voucher. The access voucher identification mechanism 12 may be an identification circuit board, for example.

The vibration unit 2 strikes the access voucher 11. Specifically, the vibration unit 2, by means of a rotation motion of the rotary electromagnet 21, drives a rotating shaft 22 and a striking head 23 to rotate, and the striking head 23 strikes a surface of the access voucher 11 so that hazardous chemical particles fall off from surface(s) of the access voucher 11.

As shown in Fig. 1, the vibration unit 2 includes the rotary electromagnet 21, the rotating shaft 22, the striking head 23 and the electromagnet holding bracket 24, wherein the rotary electromagnet 21 is equivalent to the driving mechanism, and the rotating shaft 22 and the striking head 23 are equivalent to the moving mechanism.

As shown in Fig. 2, when a positive current of 24V is supplied to the rotary electromagnet 21, the rotating shaft 22 rotates counterclockwise to position A (equivalent to the first limit position); when a negative current of 24V is supplied to the rotary electromagnet 21, the rotating shaft 22 rotates clockwise to position B (equivalent to the second limit position), and the striking head 21 strikes the access voucher 11 once. A control device provides one positive and negative alternate control signal to the rotary electromagnet 21, and the moving mechanism completes one striking on the access voucher 11.

The driving mechanism includes a rotary electromagnet 21, the moving mechanism includes a rotating shaft 22 and a striking head 23, the rotary electromagnet 21 drives the rotating shaft 22 and the striking head 23 to rotate, and the striking head 23 strikes the surface of the access voucher 11 to make the hazardous chemicals fall off from the surface(s) of the access voucher.

As described above, the vibration unit 2 applies vibrations to the access voucher by striking or shaking, for example, so that the hazardous chemicals attached to the surface(s) of the access voucher fall off from the access voucher 11, and as a result, the hazardous chemicals can be effectively collected. In particular, for particles of the hazardous chemicals that are not volatile, since they can easily fall off from the access voucher, a good collection effect can be achieved.

The above-described access voucher support unit can be shared by the access voucher information collection apparatus. In other words, the access voucher information collection apparatus collects information of the access voucher supported by the access voucher support unit. With the access voucher information collection apparatus and the hazardous chemical collection apparatus sharing a part of the structure, the hazardous chemical collection apparatus can cooperate in structure with the access voucher identification device, such as the access voucher identification mechanism, and thus the hazardous chemical collection apparatus and the access voucher information collection apparatus can be easily integrated together, which can make the structure more compact and facilitate the miniaturization of the equipment. Further, by the operation, for example, inserting the access voucher into a card reader, the access voucher information collection and hazardous chemical collection can be started at the same time easily.

Further, in the Embodiment 1, the access voucher is taken as the object under detection, and thus it is unnecessary to collect hazardous chemicals from the human body itself. Compared with the collecting method of wiping with test paper, the hazardous chemical collection process can be fully automatic, without the manual operation of security inspectors and passing persons, which can reduce the workload of personnel and reduce the labor cost; meanwhile, it can improve the collection efficiency and passing experience of the passing persons.

Furthermore, since it is unnecessary to collect hazardous chemicals by visible means such as test paper, the hazardous chemicals can be collected without being perceived by the passing person, that is, in an insensible state, and thus it does not tend to attract the attention of the suspicious persons, thereby being conducive to capturing drug carriers and other suspicious persons. In other words, the insensible detection has a better effect.

### (Embodiment 2)

In Embodiment 2, in addition to the same structure as in Embodiment 1, the hazardous chemical collection apparatus further includes: an air pump 7, an air blowing pipe 4 and an air suction pipe 5, the air pump 7 is connected with the air blowing pipe 4 and the air suction pipe 5, and can provide blowing force and suction force to the air blowing pipe 4 and the air suction pipe 5 respectively. Alternatively, there may be two air pumps 7, which are respectively connected with the air blowing pipe 4 and the air suction pipe 5. An air blowing port of the air blowing pipe 4 and an air suction port of the air suction pipe 5 can be arranged opposite each other.

The air blowing port of the air blowing pipe 4 is arranged above the vibration unit 2 and the access voucher information collection apparatus 13, preferably at a position that does not interfere with the movement of the vibration unit 2 (for example, outside the first limit position). The diameter of the air blowing port is preferably larger than the diameter of the air blowing pipe 4.

The suction port of the air suction pipe 5 is arranged below the vibration unit 2 and the access voucher information collection apparatus 13, and the air suction pipe 5 is connected with a bottom of the collection unit 3. The air blown out from the air blowing pipe 4 flows through the vibration unit 2 and the access voucher disposed in the access voucher information collection apparatus 13, and enters the air suction pipe 5. The air suction pipe 5 is connected with the hazardous chemical detection device 6 so as to transmit the collected hazardous chemicals to the hazardous chemical detection device 6.

The air suction pipe is provided with a three-way connector 51, one end of which is connected to the bottom of the collection unit 3, one end is connected to the hazardous chemical detection device 6, and the remaining end is connected to the air pump 7. The three-way connector is preferably a T-shaped three-way connector.

In the Embodiment 2, not only vibrations are applied to the access voucher, but also the air blowing and suction are applied to the access voucher. Thus, compared with Embodiment 1, which only applies vibrations, the collection amount of the hazardous chemicals can be increased, thus reducing the requirements for the detection sensitivity of the hazardous chemical detection device 6 and improving the detection accuracy.

In Embodiment 2, the hazardous chemical collection apparatus can further include two silencers. The two silencers can be respectively connected with the air blowing pipe 4 and the air suction pipe 5 to reduce the noise during air suction and air blowing, thereby avoiding the suspicious persons from finding that the hazardous chemicals are being detected and improving the effect of insensible detection.

The hazardous chemical detection device 6 is connected with the hazardous chemical collection apparatus, receives the hazardous chemicals collected by the hazardous chemical collection apparatus, and detects them. The hazardous chemical detection device 6 may include at least one of an inflammable and explosive substance detection module, a drug detection module and a toxic gas detection module. Specifically, a trace detector can be used to implement the inflammable and explosive detection module and the drug detection module. The trace detector can detect inflammable and explosive substances and drugs. The detection module of the hazardous chemical detection device 6 can be set according to the type of hazardous chemicals to be collected.

The hazardous chemical collection apparatus of the present disclosure can be installed on a gate apparatus. In other words, the present disclosure can further provide a gate system. The gate system can include: a gate apparatus including a gate and a hazardous chemical collection apparatus, and the opening and closing of the gate can be controlled by the detection results of hazardous chemicals collected by the hazardous chemical collection apparatus. The gate system can be a single gate structure with one gate, or a double gate structure with two gates at the front and back. In the case of the single gate structure, the gate is controlled by the detection results of the hazardous chemicals. In the case of the double gate structure, the hazardous chemicals can be collected outside the gate at the entrance side, and the opening and closing of the gate at the entrance side can be controlled according to whether the collection is completed, while the opening and closing of the gate at the exit side can be controlled by the detection results of the hazardous chemicals.

The gate system can further include: an access voucher information collection apparatus, which is installed on the gate apparatus and collects access voucher information of the access voucher supported by the access voucher support unit; and the hazardous chemical collection apparatus, which is installed on the gate apparatus and applies vibrations to the access voucher supported by the access voucher support unit so as to collect hazardous chemicals. That is, in the gate system, the access voucher information collection apparatus and the hazardous chemical collection apparatus share the access voucher support unit, and thus can be integrated together and installed on the gate apparatus. The installation position of the access voucher information collection apparatus and the hazardous chemical collection apparatus is preferably the part of the gate apparatus at the entrance side of the gate. Thus, hazardous chemical collection function can be integrated to the gate apparatus of the gate system. Moreover, the hazardous chemicals can be collected outside the gate in parallel with the access voucher information collection, thereby reducing the passage time of the gate apparatus.

Although the present disclosure has been described with reference to exemplary embodiments, the person skilled in the art can make various modifications and variations without departing from the spirit and scope of the present disclosure, and such modifications and variations fall within the scope defined by the claims.

## Claims

1. A hazardous chemical collection apparatus, comprising:
a vibration unit and a collection unit,
wherein the vibration unit applies vibrations to an access voucher to cause hazardous chemicals attached to a surface of the access voucher to fall off, and
the collection unit is located below the access voucher to collect the hazardous chemicals falling off from the access voucher.

2. The hazardous chemical collection apparatus according to claim 1, wherein the vibration unit comprises a driving mechanism and a moving mechanism, and
the driving mechanism is connected with the moving mechanism and drives the moving mechanism to move between a first limit position away from the access voucher and a second limit position close to the access voucher, so as to apply vibrations to the access voucher.

3. The hazardous chemical collection apparatus according to claim 1, further comprising an access voucher support unit,
wherein the access voucher support unit is configured to support the access voucher, and
the vibration unit applies vibrations to the access voucher supported by the access voucher support unit.

4. The hazardous chemical collection apparatus according to claim 3, wherein the access voucher support unit further comprises an access voucher identification mechanism identifying the access voucher.

5. The hazardous chemical collection apparatus according to claim 3, wherein relative position of the vibration unit and the access voucher support unit is stationary.

6. The hazardous chemical collection apparatus according to claim 2, wherein the driving mechanism drives the moving mechanism to perform translation or rotation movement between the first limit position and the second limit position.

7. The hazardous chemical collection apparatus according to claim 2, wherein the moving mechanism comprises a striking portion,
wherein when the driving mechanism drives the striking portion to move from the first limit position to the second limit position, the striking portion strikes a surface of the access voucher.

8. The hazardous chemical collection apparatus according to claim 2, wherein the moving mechanism comprises a shaking portion capable of clamping the access voucher, and
when the driving mechanism drives the shaking portion to move back and forth between the first limit position and the second limit position, the access voucher is shaken.

9. The hazardous chemical collection apparatus according to claim 6, wherein the driving mechanism comprises a rotary motor or a rotary electromagnet,
the moving mechanism comprises a rotating shaft and a striking head,
the rotating shaft is connected with the driving mechanism, and the striking head is connected with the rotating shaft, and
when the driving mechanism rotates, it drives the rotating shaft to rotate, and through the rotation of the rotating shaft, the striking head rotates between the first limit position and the second limit position and strikes a surface of the access voucher.

10. The hazardous chemical collection apparatus according to claim 1, wherein a longitudinal section of the collection unit has a large upper portion and a small lower portion.

11. The hazardous chemical collection apparatus according to claim 3, further comprises an air blowing pipe and an air suction pipe,
wherein an air blowing port of the air blowing pipe is disposed above the access voucher support unit,
the air suction pipe is connected with a bottom of the collection unit, and
air blown out from the air blowing pipe flows across the access voucher supported by the access voucher support unit and enters the air suction pipe through the collection unit.

12. The hazardous chemical collection apparatus according to claim 11, wherein the air suction pipe is installed with a three-way connector, and the air suction pipe is respectively connected with the bottom of the collection unit and a hazardous chemical detection device through the three-way connector.

13. The hazardous chemical collection apparatus according to claim 11, further comprises two silencers, one of the two silencers is connected with the air blowing pipe, and another one of the two silencers is connected with the air suction pipe.

14. A gate system, comprising:
the hazardous chemical collection apparatus according to any one of the claims 1 to 13.

15. The gate system according to claim 14, further comprises:
a gate apparatus, comprising a gate; and
an access voucher information collection apparatus, comprising an access voucher support unit, the access voucher information collection apparatus collecting access voucher information from an access voucher supported by the access voucher support unit,
wherein the hazardous chemical collection apparatus shares the access voucher support unit with the access voucher information collection apparatus, and applies vibrations to the access voucher supported by the access voucher support unit to collect hazardous chemicals, and
the access voucher information collection apparatus and the hazardous chemical collection apparatus are installed on an outer side of the gate in the gate apparatus.
